# EUROPEAN PATENT SPECIFICATION

(11) **EP 4 192 550 B1**
(45) Date of publication and mention of the grant of the patent: **06.05.2026**
(21) Application number: 21758072.9
(22) Date of filing: 02.08.2021
(51) Int. Cl.: A61M 5/31, A61M 5/315

(54) **DRUG DELIVERY DEVICE AND MODULE HEREWITH**
ARZNEIMITTELABGABEVORRICHTUNG UND MODUL
DISPOSITIF D'ADMINISTRATION DE MÉDICAMENTS ET MODULE ASSOCIÉ

(30) Priority: 04.08.2020 EP 20315377
(43) Date of publication of application: 14.06.2023
(73) Proprietor: Sanofi, 75017 Paris (FR)
(72) Inventor: GAZELEY, Oliver, Charles, 4058 Basel (CH); TOPOREK, Maurice, 65926 Frankfurt am Main (DE)
(74) Representative: Keil & Schaafhausen Patentanwälte PartGmbB
(86) International application number: PCT/EP2021/071518
(87) International publication number: WO 2022/029052

(56) References cited:
- WO-A1-2014/111335
- WO-A1-2014/111341
- WO-A1-2014/111343
- WO-A1-2017/016959
- WO-A1-2017/081051
- WO-A1-2017/186841
- WO-A2-2010/052275

## Description

The present invention is generally directed to a drug delivery device. The present invention further relates to a module, e.g. an add-on electronic device, to be used in conjunction with a drug delivery device.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is generally applicable for disposable and reusable devices. However, the present invention is especially applicable in pre-filled, disposable pen type devices.

Such drug delivery devices typically comprise dose setting and/or a drive mechanism to select an individual dose and to deliver this dose by displacing the piston in a cartridge containing a medicament.

It is important for patients to be able to precisely select (dial) a desired dose prior to dispensing, thereby avoiding underdosage or overdosage which may result in severe health problems. For this purpose, it is known to provide a display on which the actually selected dose is indicated. In addition, a tactile or audible feedback may be generated, for example a click sound for each dose unit dialed. WO 2010/139636 A1 and EP 2 890 434 B1 each disclose such a drug delivery device having a display and a feedback mechanism comprising a detent or clicker which provides the user with an audible and tactile feedback as a dose is set.

In addition to providing a feedback or indication relating to the currently selected dose to the patient, it is known to track and manage data relating to the type and/or amount of drug administered. For example, WO 2019/101962 A1, WO 2019/040117 A1 and WO 2020/127006 A each propose an electronic module to detect a movement of a component part of a drug delivery device which movement may be indicative for a state of the device or a quantity of a selected (dialed) or dispensed dose. These modules are integrated into the respective drug delivery device which raises the costs for manufacturing the devices. Thus, these modules are typically applicable in re-usable devices.

WO 2014/111341 A1, WO 2014/111343 A1 and WO 2014/111335 A1 disclose similar electronic modules suitable for use wth a drug delivery device, wherein e.g. WO 2014/111341 A1 proposes a module detachable from the drug delivery device. In these devices, an electrically conductive track may be provided on a component part of the drug delivery device, e.g. on the number sleeve.

In addition, it is known to use separate modules which may be clamped onto the drug delivery device and use contactless technologies to detect a movement of a component part of a drug delivery device.

It is an object of the present invention to provide an improved drug delivery device allowing reliable and cost-effective detection of the selected dose. It is a further object to provide a module for use with such a drug delivery device.

This object is solved for example by the subject matter defined in the independent claims. Advantageous embodiments and refinements are subject to the dependent claims. However, it should be noted that the disclosure is not restricted to the subject matter defined in the appended claims. Rather, the disclosure may comprise improvements in addition or as an alternative to the ones defined in the independent claims as will become apparent from the following description.

An example of a drug delivery device comprises a dose setting and driving assembly for dialing and dispensing a dose of a medicament from a cartridge of the drug delivery device. Preferably, the dose setting and driving assembly comprises an outer housing, a number sleeve and a dose dial grip, wherein a portion of the number sleeve is visible through an aperture or window in the outer housing to display the actually dialed dose. The number sleeve and the dose dial grip may be rotatable relative to the outer housing during dose dialing and the number sleeve may be rotatable relative to the outer housing during dose dispensing, while the dose dial grip is axially displaceable and rotationally constrained to the outer housing during dose dispensing. The dose dial grip may comprise an outer skirt partially protruding out of the outer housing. Preferably, the outer skirt comprises a profiled gripping surface at or near the proximal end of the dose dial grip.

One aspect of the disclosure relates to the application of dial encoding to an electronic re-usable clip-on module. Dial encoding carries potential advantages for users, as the electronic system is able to detect what doses are being selected before any insulin is delivered to a patient. A re-usable electronic module to achieve this is more cost effective than embedding costly electronics in every drug delivery device. In other words, according to one aspect of the present disclosure, the detection of a selected (dialed) dose is achieved by, preferably low-cost, component parts embedded in a drug delivery device which may be a disposable device and component parts of a detecting module which may be, e.g. releasably, attached to such a drug delivery device.

According to a further aspect of the present disclosure, the drug delivery device may be fully operational without the detection module such that the module, if used with the device only adds a tracking or detecting function.

In still another aspect of the present disclosure, drug delivery device, especially the outer housing, the number sleeve and the skirt of the dose dial grip, can be void of any active electronic components. It may comprise only passive electrically conducting components, such as conductive strips, electric contact tabs or at least one bridging contact.

In one example, at least one of the number sleeve and the skirt of the dose dial grip is provided with a series of circumferentially spaced conductive strips distributed around the outer circumference of the number sleeve or the skirt, and a series of contacts is arranged within the outer housing biased to abut the conductive strips. The strips and the contacts are preferably arranged such to form a detector arrangement, e.g. an encoder, for detecting relative movement between the number sleeve and/or the skirt and the outer housing. According to the invention, the conductive strips may provice at least two different pattern portions which are electrically separated, e.g. along a circumference or along a tangential direction of a tubular-shaped surface of the number sleeve and/or skirt of the drug delivery device. According to the invention, the series of contacts may be part of at least one bridging contact which may be provided in the drug delivery device configured and operable to selectively establish an electric connection between at least two different pattern portions as number sleeve and/or skirt is subject to a helical or rotational movement relative to the outer housing. In other words, a rotary encoder may be provided embedded in the drug delivery device by providing a bridging contact (formed by the series of contacts) in combination with at least two different electrically conductive pattern portions (formed by the conductive strips) which are electrically separated.

To allow easy connection with an add-on module, the conductive strips and/or the contacts are, according to the invention, directly accessible from outside the outer housing. In other words, an electric contact between the module and the detector arrangement of the drug delivery device may be established without having to remove parts of the drug delivery device as the conductive strips and/or the contacts are directly accessible from outside the outer housing. As an alternative, it may be required to remove a cap from the drug delivery device to be able to fit the module onto the drug delivery device, e.g. on the dose dial grip.

For example, the series of contacts may form at least one reference element and the conductive strips may form a pattern of an encoder assembly. In other words, the contacts and the conductive strips together may form a detector arrangement for at least one electric sensor being operable to detect a positional variation of the pattern relative to the at least one reference element and to generate at least one electric signal in response to the positional variation of the pattern during at least one of the relative movements of the number sleeve and/or the skirt during dose dialing and dose dispensing. The at least one sensor itself may comprise, in addition to these passive electronic components of the drug delivery device, active electronic components. Preferably, the encoder assembly is based on the working principle as disclosed in WO 2020/127006 A to which reference is made regarding the disclosure of the encoder functions.

In an example of the present disclosure, the pattern comprises at least a first pattern portion that is electrically conductive. The pattern comprises at least a second pattern portion that is electrically insulating. Typically, the pattern comprises numerous first pattern portions and numerous second pattern portions. For instance, the pattern comprises a sequence of electrically conductive portions and a sequence of electrically insulating portions. The electrically conductive pattern portions may be electrically separated or galvanically separated from each other through the electrically insulating second pattern portions. The first pattern portion and the second pattern portion being electrically conductive and electrically insulating, respectively may correspond and represent the above mentioned first and second pattern portions of the pattern that are arranged non-overlapping with respect to each other and that distinguish with regard to their electrical conductivity.

A cost effective and simple way to provide the conductive strips forming the pattern is to print the conductive strips onto a component of the drug delivery device. For example, the conductive strips are printed on the skirt of the dose dial grip. As an alternative, the conductive strips are provided on an encoder wheel fixed to or provided on the number sleeve. When the pattern comprises an electrically conductive structure, e.g. when the first pattern portion is electrically conductive it may comprise one of a conductive varnish, a conductive lacquer, a conductive coating or conductive etching. The conductive varnish or conductive lacquer may comprise electrically conductive particles, such as metal particles or carbon black particles. The electrically conductive pattern may also comprise a metal inlay in or on the tubular-shaped surface of the number sleeve and/or skirt. The number sleeve and/or skirt may comprise a thermoplastic material being substantially electrically insulating. In this way only the electrically conductive pattern portions have to be provided on the electrically insulating material of the number sleeve and/or skirt. Still further, the electrically conductive first pattern portion may also comprise a sheet metal attached to or embedded in and flush with the tubular-shaped surface of the number sleeve and/or skirt. The at least first electrically conductive pattern portion may be attached or assembled to the tubular-shaped surface by way of insert molding or by way of a two-component injection molding of the number sleeve and/or skirt.

The first and second pattern portions may be arranged alternately along at least one of the movements between the number sleeve and/or skirt and the outer housing. The first and the second pattern portions may be separated along a circumference or along a tangential direction of the tubular-shaped surface of the number sleeve and/or skirt. The first and the second pattern portions may also be separated along the longitudinal direction of the tubular-shaped surface.

The first and the second pattern portions may comprise a stripe pattern with an alternating arrangement of stripes exhibiting at least two different electrical conductivities. The pattern is not limited to a first pattern section and to a second pattern section. There may be provided numerous different pattern sections, such as first, second, third or even more pattern sections that all distinguish from each other e.g. with regard to their electrical conductivity. In this way a higher information density or code density can be provided on the tubular-shaped surface.

In some examples the pattern is provided on an outside surface of the number sleeve and/or skirt. In other examples the pattern is provided on an inside surface of the number sleeve and/or skirt. The pattern may comprise a binary pattern comprising an information content provided by at least two, namely first and second pattern portions representing a digital 0 or a digital 1, respectively. The pattern and the at least one electric sensor may be implemented as an incremental or as an absolute quadrature encoder. They may be implemented as a 2-bit gray code. Depending on the number of electric sensors or electric contact tabs and depending on the specific implementation of the pattern also other codes, comprising a 3-bit encoding or an n-bit encoding with n being an integer number can be provided.

In a further example the detector arrangement comprises at least one tap as the electrical contact arranged on the outer housing and operable to alternately connect to the first pattern portion and the second pattern portion of the pattern when the number sleeve and/or skirt is subject to a movement relative to the outer housing. The electrical contact tap may be radially biased so as to frictionally engage with the pattern of the number sleeve and/or skirt. When the pattern is provided on an outer tubular-shaped surface of the number sleeve and/or skirt the at least one electrical contact tap is biased radially inwardly. It is flexible or deformable radially outwardly by the pattern against an inherent restoring force.

When the tubular-shaped surface is an inner surface the at least one electrical contact tap is biased radially outwardly and can be deformed or flexed radially inwardly against a respective restoring force. The electrical contact tap may represent the reference element. Hence, the reference element may comprise the at least one electrical contact tap. The reference element may comprise numerous electrical contact tabs arranged and distributed along the outer or inner circumference of the tubular-shaped surface.

In another example the pattern comprises at least a third pattern portion that is electrically conductive, wherein the first pattern portion and the third pattern portion are electrically separated from each other. Hence, the first pattern portion and the third pattern portion are galvanically insulated from each other. The first pattern portion and the third pattern portion can be electrically separated by the second pattern portion that is electrically insulating. Providing at least two different types of electrically conductive pattern portions allows and supports implementation of an n-bit rotary encoder, with n being an integer equal to or larger than 2. When the first pattern portion and the third pattern portion are electrically distinguishable they can be individually and/or separately electrically connectable to the detector arrangement when subject to one of the first and second movement thus enabling e.g. a 3-bit rotary encoder.

In other examples the third pattern portion, when used together with an electrical add-on module, may be permanently electrically connected to a voltage supply of the detector arrangement. Here, the at least one electric sensor may be electrically connected to the first pattern portion. The rotary encoder may be completed by a bridging contact provided on the outer housing. The bridging contact may be configured and operable to selectively establish an electric connection between the third pattern portion and the first pattern portion as number sleeve and/or skirt is subject to a helical or rotational movement relative to the outer housing.

In another example the detector arrangement and the at least one electric sensor are arranged on the number sleeve and/or the skirt of the dose dial grip. The at least one electric sensor is electrically connected to the first pattern portion. The at least one reference element is arranged on the outer housing and comprises an electrical bridging contact. The electrical bridging contact is configured to alternately establish and interrupt an electric contact between the first pattern portion and the third pattern portion when the number sleeve and/or the skirt of the dose dial grip is subject to a movement relative to the outer housing.

Typically, the electrical bridging contact extends in a tangential (circumferential) direction with regard to the tubular shape of the number sleeve and/or the skirt. The bridging contact may be implemented and operable to establish an electrical contact between the first pattern portion and the third pattern portion, e.g. thereby bridging the second pattern portion located between and/or separating the first pattern portion and the third pattern portion. This electric connection between the first pattern portion and the third pattern portion is typically provided when the number sleeve and/or the skirt is in a first rotational position or rotational orientation with regard to the outer housing. As the number sleeve and/or the skirt is subject to a further rotation and arrives at a second rotational state or rotational orientation at least one of the first pattern portion and the third pattern portion loses contact with the electrical bridging contact. In this way, the first pattern portion and the third pattern portion become electrically or galvanically separated. With the module mounted to the drug delivery device, since the at least one electric sensor is electrically connected to the first pattern portion a varying electrical contact with the third pattern portion can be detected by the at least one electric sensor as the number sleeve and/or the skirt is subject to a rotation relative to the housing. Here, the third pattern portion may also be electrically connected to the at least one electric sensor or to another electric sensor of the detector arrangement. Alternatively, the third pattern portion may be permanently connected to a voltage supply of the detector arrangement, when the module is attached. With rotational positions of the number sleeve and/or the skirt and the outer housing, in which the first pattern portion is electrically connected to the third pattern portion via the at least one electrical bridging contact the number sleeve and/or the skirt is provided with the supply voltage. In other rotational states, wherein the first pattern portion is electrically separated from the third pattern portion the electric sensor connected to the first pattern portion will detect a zero voltage.

Use and implementation of at least one electrical bridging contact is beneficial because the outer housing can be easily adapted for the implementation and embedding of the detector arrangement. Here, only the geometry of the outer housing has to be slightly modified in order to receive or to assemble the at least one electrical bridging contact. Insofar, the outer housing does not require any active electric or electronic components but requires only a passive electrically conductive structure.

With another example the electrical bridging contact comprises a first electrical contact tap and a second electrical contact tap. The first electrical contact tap and the second electrical contact tabs are electrically connected. The first electrical contact tap and the second electrical contact tap are spatially separated from each other along a first separation direction parallel to a distance between the first pattern portion and the third pattern portion. The magnitude of spatial separation between the first electrical contact tap and the second electrical contact tap is typically larger than a size or width of at least one of the first pattern portion and the third pattern portion as seen along the first separation direction.

Typically, the spatial separation between the first electrical contact tap and the second electrical contact tap is at least equal to or larger than the size or extension of the second pattern portion located between the first pattern portion and the third pattern portion as seen along the first separation direction. In this way it is ensured, that at least with one rotational state or rotational orientation of the number sleeve and/or the skirt relative to the outer housing the first pattern portion may be electrically connected to the third pattern portion via the electrical bridging contact, namely when the first electrical contact tap is in electrical connection with the first pattern portion and the second electrical contact tap is in electrical connection with the third pattern portion.

In another example the first separation direction extends substantially parallel to an imaginary shortest connection between the first pattern portion and the third pattern portion. For instance, if the first and the second pattern portions are portions of a stripe pattern the first separation direction extends substantially perpendicular to the longitudinal extension of the stripes of the pattern.

According to a further example the electrical bridging contact comprises a third electrical contact tap spatially separated from at least one of the first electrical contact tap and the second electrical contact tap along a second separation direction that is non-parallel to the first separation direction. With some examples the second separation direction extends substantially perpendicular to the first separation direction. If the pattern on the first element comprises a longitudinal stripe pattern the first and second electrical contact tabs are particularly configured to alternately engage or to alternately contact the alternating stripes as the number sleeve and/or the skirt is subject to a rotation or helical movement relative to the outer housing. Typically, the first and the second electrical contact tabs are only displaceable relative to the number sleeve and/or the skirt in the region of the first pattern section. Moreover, the first pattern section may be entirely provided with a stripe pattern comprising at least one first, second and at least one third pattern portion.

The third electrical contact tap of the electrical bridging contact may be located offset from at least one or from both of the first electrical contact tap and the second electrical contact tap along the longitudinal direction. In this way and as the number sleeve and/or the skirt is subject to a longitudinal displacement relative to the outer housing the third electrical contact tap may reach into or onto the second pattern section. The second pattern section may be separated from the first pattern section along the longitudinal direction. The second pattern section may be out of reach for the first and the second electrical contact tabs of the electrical bridging contact but it may be engageable with only the third electrical contact tap of the electrical bridging contact. In this way the interaction of the third electrical bridging contact with the second pattern section may be an indicator that a longitudinal end position, e.g. a zero dose configuration or end-of-dose configuration of the injection device has been reached.

Accordingly, and in another example the third bridging contact is configured to get in contact with the second pattern section when the number sleeve and/or the skirt and the outer housing return into an initial relative position after completion of the dispensing of the dose. In this way, the mutual engagement or contact between the third bridging contact and the second pattern section provides a zero dose indicator thus indicating to the detector arrangement, that the end of a dose dispensing or expelling procedure has been reached.

In further examples the drug delivery device comprises two or more electrical bridging contacts that are distributed along the tubular circumference of the number sleeve and/or the skirt. The two or more electrical bridging contacts may be located at the same longitudinal position on the outer housing. They may be equiangularly or equidistantly arranged along the circumference or along the tangential (circumferential) direction of the tubular-shaped surface of the number sleeve and/or the skirt.

Typically, also the outer housing is of tubular shape. Accordingly, the two or more electrical bridging contacts can be separated from each other along the circumference or tangential direction of the tubular-shaped outer housing. The two or more electrical bridging contacts can be implemented identically. Hence, a first electrical bridging contact has the same shape and geometry compared to a second electrical bridging contact. The two or more electrical bridging contacts can be also asymmetrically arranged along the circumference of the outer housing. Hence, a distance or angular distance between a first and a second electrical bridging contact may differ from a distance or angular distance between the second and a third electrical bridging contact. The geometric arrangement of the two or more electrical bridging contacts depends on the encoding of the pattern on the number sleeve and/or the skirt.

In another example the two or more electrical bridging contacts and the first and the third pattern portions are arranged such, that in any available rotational position of the number sleeve and/or the skirt relative to the outer housing at least one of the first pattern portions is electrically connected to at least one of the third pattern portion via the at least one of the bridging contacts. In this way, at least one of the first pattern portions is at a supply voltage if the at least one third pattern portion is connected to a voltage supply. In this way, an unequivocal electrical signal can be provided for each available rotational position of the number sleeve and/or the skirt relative to the outer housing.

The electrical bridging contact may comprise a body made of sheet-metal and comprising at least one flexible arm, wherein at least one of the first electrical contact tap and the second electrical contact tap is arranged at a free end of the at least one flexible arm.

For facilitating attachment of the module on the drug delivery device, the dose dial grip may comprise mechanical fixation means, e.g. clip features, and/or rotational alignment means for attaching a separate module on the dose dial grip. For example, the re-usable module may be mechanically dedicated to fit to the drug delivery device in the correct orientation, e.g. using poka-yoke features.

A further aspect of the present disclosure is directed to a module suitable to be used in conjunction with a drug delivery device as described above. A re-usable clip-on electronic module can be configured to fit onto the button end of the drug delivery device. Electrical connections may be made to the conductive areas using metallic contacts, which act as inputs to a microcontroller, allowing rotational de-coding. These metallic contacts are preferably designed to deflect radially outwards when the module is assembled to a drug delivery device, such that when the module is attached to the device, the metallic contacts are biased radially against the conductive areas, ensuring good electrical connection. In other words, the electrical connectors may comprise metal clips having a free end biased radially inwards for abutting the conductive strips of the drug delivery device.

In more detail, while at least one of the number sleeve and the skirt of the dose dial grip of the drug delivery device is provided with a series of circumferentially spaced conductive strips distributed around the outer circumference of the number sleeve or the skirt, and a series of contacts is arranged within the outer housing of the drug delivery device biased to abut the conductive strips, the module comprises contacts which are biased radially against the conductive areas of the drug delivery device. Thus, a re-usable electronic module may be used in combination with component parts embedded in an, e.g. disposable, drug delivery device.

A module for detecting movement of at least one component part of a drug delivery device may comprise a microprocessor for processing and/or storing data from a detector arrangement of the drug delivery device, electrical connectors, i.e. metallic contacts, to releasably establish contact with conductive strips or contacts of the drug delivery device, and mechanical fixation means, e.g. clip features, and/or rotational alignment means for attaching the module on the drug delivery device, e.g. on the dose dial grip. The module or its microprocessor may contain a rigid PCB, a coin cell, and can be configured to communicate via NFC or Bluetooth with a smart device for logging and recording insulin doses.

The module may further comprise a cap to be, e.g. releasably, fitted over the dose dial grip of the drug delivery device. The cap may comprise an outer profiled gripping surface and encloses the microprocessor and the electrical connectors.

A still further aspect of the present disclosure is directed to a drug delivery device as described above together with a module as described above.

The present invention is applicable for devices which are manually driven, e.g. by a user applying a force to an injection button, for devices which are driven by a spring or the like and for devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

The drug delivery device may comprise a cartridge containing a medicament. The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

Non-limiting, exemplary embodiments of the invention will now be described with reference to the accompanying drawings, in which:
- Figure 1: shows a view of a drug delivery device according to a first embodiment of the invention without a module;
- Figure 2: shows the drug delivery device of Figure 1 with a module attached;
- Figure 3: shows a view of a dose dial grip of the drug delivery device of Figures 1 and 2;
- Figure 4: shows a view of the proximal end of the drug delivery device of Figures 1 and 2 with the dose dial grip and number sleeve removed;
- Figure 5: shows a sectional view of the module of Figure 2;
- Figure 6: shows a sectional view of the drug delivery device of Figures 1 and 2 with the module attached;
- Figure 7: shows a sectional view of a proximal part of a drug delivery device according to a second embodiment of the invention; and;
- Figure 8: shows a perspective partially cut away view on the drug delivery device of Figure 7.

In the Figures, identical elements, identically acting elements or elements of the same kind may be provided with the same reference numerals.

The terms "axial", "radial", or "circumferential" as used herein may be used with respect to a main longitudinal axis I of the device, the cartridge, the housing or the cartridge holder, e.g. the axis which extends through the proximal and distal ends of the cartridge, the cartridge holder or the drug delivery device.

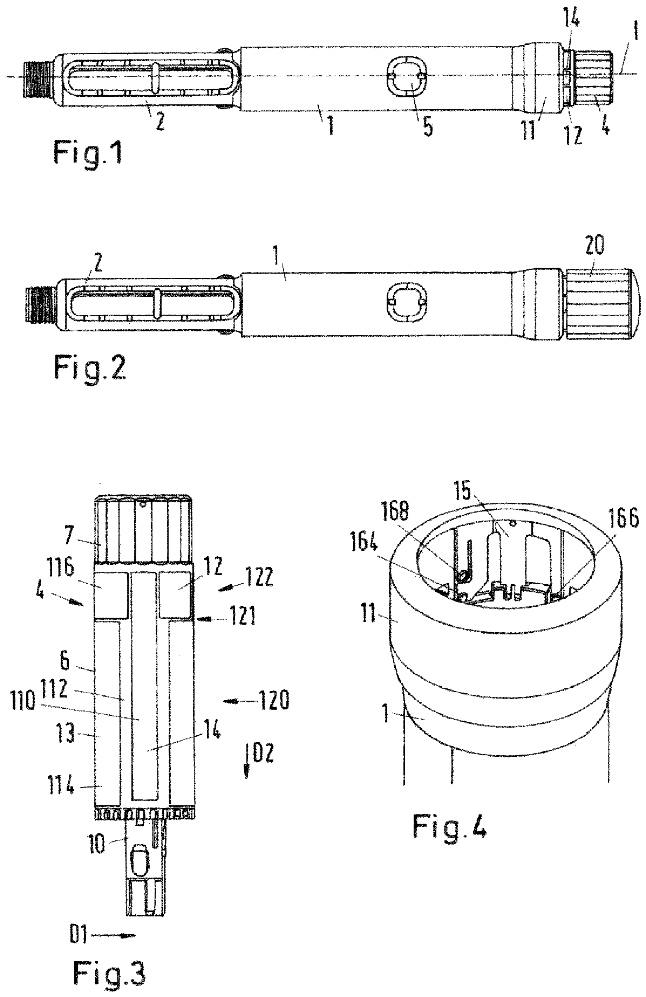

"Distal" is used herein to specify directions, ends or surfaces which are arranged or are to be arranged to face or point towards a dispensing end of the drug delivery device, i.e. the left side in Figures 1 and 2, or components thereof and/or point away from, are to be arranged to face away from or face away from the proximal end, i.e. the right end in Figures 1 and 2. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

A first embodiment of a drug delivery device of the invention is depicted in Figures 1 to 6. The drug delivery device comprises an outer housing 1 and a cartridge holder 2 for retaining a cartridge comprising medicament. The cartridge holder 2 may be permanently or releasably attached to the outer housing 1.

The outer housing 1 is part of a dose setting and driving assembly further comprising at least a number sleeve 3 and a dose dial grip 4. A portion of the number sleeve 3 is visible through window 5 in the outer housing 1, thus providing a dose display. The dose dial grip 4 comprises an outer skirt 6 partially protruding out of the proximal end of the outer housing 1 and comprising a profiled gripping surface 7 at the proximal end of the dose dial grip 4. Preferably, the dose dial grip 4 is suitable for two different types of actuation, e.g. a rotation about the longitudinal axis I of the drug delivery device and a displacement parallel to the longitudinal axis I of the drug delivery device.

The dose setting and driving assembly may comprise further component parts, for example a driver 8 for driving a piston rod (not shown), a clutch 9 for coupling and decoupling the driver 8 and the number sleeve 3 and/or of the dose dial grip 4 to switch between a dose setting mode (dose dialing) of the drug delivery device and dose dispensing mode, and an inner housing (not shown). For example, the number sleeve 3 and the dose dial grip 4 may be rotatable relative to the outer housing 1 during dose dialing and the number sleeve 3 may be rotatable relative to the outer housing 1 during dose dispensing, while the dose dial grip 4 is axially displaceable and rotationally constrained to the outer housing 1 during dose dispensing. The number sleeve 3 may be in threaded engagement with a stationary component part, e.g. the inner housing, such that the rotation of the number sleeve 3 results in a helical displacement, i.e. a rotational movement combined with an axial movement. In the embodiment depicted in the Figures, the number sleeve 3 comprises a ring of radially outwards protruding clutch teeth 9 near its proximal end for engaging corresponding radially inwards protruding clutch teeth provided on the dose dial grip 4. This clutch 9 may be biased into engagement of the respective clutch teeth which may be disengaged by displacing the dose dial grip 4 relative to the number sleeve 3 by exerting a force on the proximal end face of the dose dial grip 4. In other words, with the clutch 9 engaged or coupled, the number sleeve 3 follows a rotation of the dose dial grip 4 upon actuation of a user. Further, with the clutch 9 disengaged or decoupled, the dose dial grip 4 may be displaced axially without rotation, while the number sleeve 3 follows this axial movement and is forced by the threaded engagement with the stationary component part to move on the helical path.

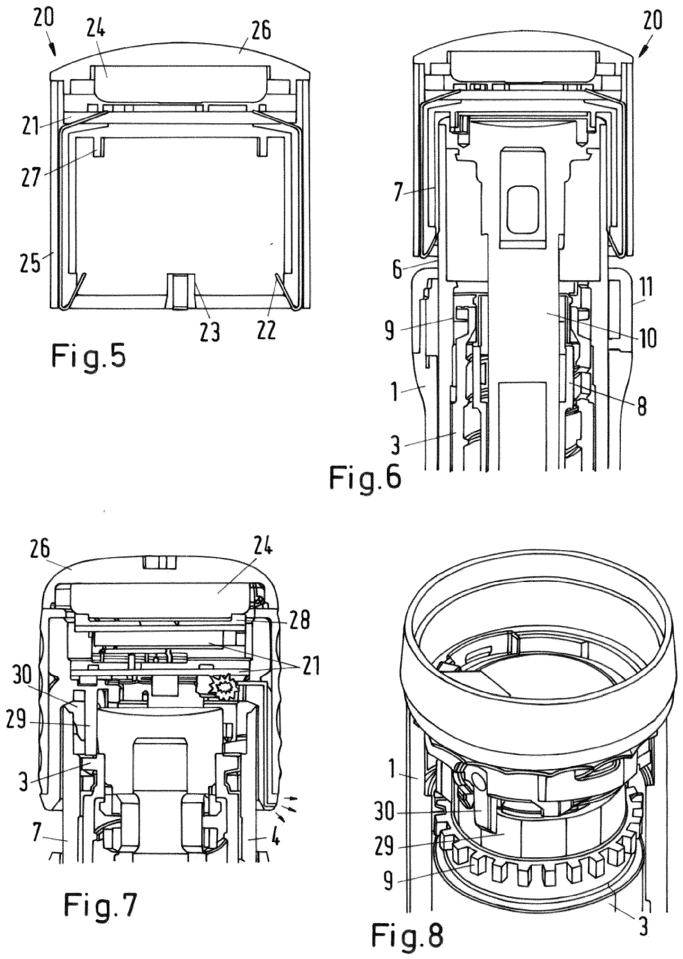

As shown in Figures 3 and 6 in more detail, the dose dial grip 4 may further comprise an inner stem 10 which is permanently constrained to the skirt 6 and the profiled gripping surface 7 in the depicted embodiment. As an alternative, the inner stem 10 may be integrally formed with the skirt 6 and the profiled gripping surface 7.

Further, the outer housing 1 may comprise a retention cap 11 which is permanently attached to the proximal end of the outer housing 1. The retention cap 11 may have the form of a ring or of the sleeve as shown in Figure 4. Thus, the retention cap 11 and covers a portion of the dose dial grip 4. As an alternative to the retention cap 11 being a separate component part, the retention cap may be a unitary portion of the outer housing 1.

As shown in Figure 3 in more detail, the skirt 6 of the dose dial grip 4 is provided with a series of electrically conductive strips 12, 13, 14 which may be printed, plated or etched on the outer cylindrical surface defined by the skirt 6. The electrically conductive strips are located space from the profiled gripping surface 7 of the dose dial grip 4 in the embodiment depicted in Figures 1 to 6. At least a portion of the strips 12 and 14 protrude from the proximal end of the outer housing 1 and/or of the retention cap 11 in the states depicted in Figures 1 and 2 in which the number sleeve 3 is in its most distal position, i.e. a position in which no dose is selected (dose size zero). As a higher dose is dialed by rotation of the dose dial grip 4 and the number sleeve 3, the strips 12 and 14 protrude further from the proximal end of the outer housing 1 and/or the retention cap 11. In addition, the strips 13 may protrude from the proximal end of the outer housing 1 and/or the retention cap 11 as a dose larger than zero is dialed. These conductive areas 12, 13, 14 comprise a 'Live' area, two 'Sensor' areas and a '0U switch' area, forming a 2-bit rotational quadrature encoder with end-of-dose 0U switch.

Further, an electrically conductive element 15 comprising a series of contacts (contact tabs) 164, 166 and 168 is arranged within the outer housing 1 and/or the retention cap 11 to form bridging contacts as a reference. In the depicted embodiment the conductive element 15 is fully retained within the outer housing 1 and/or the retention cap 11. The series of contacts is arranged within the outer housing 1 biased to abut the conductive strips 12, 13, 14. The conductive strips and the conductive element 15 with its contact tabs form of an encoder assembly for recording doses that are dialled and delivered from the drug delivery device. The conductive bridging contacts or tabs of element 15 are mounted within the device housing 1 to alternately couple and de-couple 'Live' strips to 'Sensor' strips as the dose dial grip 4 rotates. These bridging contacts are not electrically connected to the module which will be explained below or its PCB, and act only to provide a conductive path between adjacent strips 12, 13, 14 on the skirt 6. The bridging contacts are rotationally and axially aligned within the outer housing 1 and can be retained with the retention cap 11. Preferably, the encoder assembly is based on the working principle as disclosed in WO 2020/127006 Ato which reference is made regarding the disclosure of the encoder functions.

The module 20 for detecting movement of at least one component part of the drug delivery device, is depicted in Figures 5 and 6. In Figure 6, the module 20 is attached to the drug delivery device, specifically to the proximal end of the dose dial grip 4 and may comprise an outer profiled gripping surface.

In the depicted example, the module 20 comprises a microprocessor 21 for processing and/or storing data from a detector arrangement of the drug delivery device. Further, the module 20 comprises electrical connectors 22 to releasably establish contact with at least some of conductive strips 12, 13, 14 of the drug delivery device and mechanical fixation means, e.g. clip features 23, for attaching the module 20 on the drug delivery device. The module 20 further comprises a power supply for the microprocessor 21, for example in the form of a coin cell 24. The microprocessor 21, the connectors 22 and the coin cell 24 are received in a cap-like housing 25 which may be provided with a separate end cap 26 as depicted in Figures 5 and 6. As an alternative, the end cap 26 may be a unitary part with the housing 25. In addition to the clip features 23, rotational alignment means 27 may be provided in or on the housing 25 for aligning the housing 25 mechanically with the dose dial grip 4, specifically the profiled gripping surface 7. The module 20 may be mechanically dedicated to fit to the drug delivery device in the correct orientation using poka-yoke features, which may be alignment features 27.

The module 20 is preferably a re-usable clip-on module configured to fit onto the dose dial grip 4. Electrical connections are made to at least some of the conductive strips 12, 13, 14 of the skirt 6 using metallic contacts of the connectors 22, which act as inputs to a micro-controller, allowing rotational de-coding. These metallic contacts of the connectors 22 are designed to deflect radially outwards when the module 20 is assembled to the drug delivery device, such that when the module is attached to the device, the metallic contacts are biased radially against the conductive areas formed by the conductive strips 12, 13, 14 of the skirt 6, ensuring good electrical connection.

The microprocessor 21 of the module 20 may be provided on a rigid PCB and can be configured to communicate via NFC or Bluetooth with a smart device for logging and recording insulin doses dialled with and/or dispensed from the drug delivery device.

In the following, the detection of movements in the drug delivery device by means of the module 20 is described in more detail.

On the outer surface of the tubular-shaped skirt 6, at least one pattern is provided by the electrically conductive strips 12, 13, 14. The pattern comprises a first pattern section 120, a separation 121 and a second pattern section 122. The first pattern section 120 extends almost over the entirety of the longitudinal elongation of the skirt 6 of the dose member 4. The second pattern section 122 is located at or near a proximal end of the skirt 6. The pattern comprises numerous pattern portions 110, 112, 114, 116. The pattern portions 110 are formed by the conductive strips 14 and are electrically conductive, while the pattern portions 112 are electrically insulating. The pattern portions 110, 112 comprise an elongated shape mainly extending parallel to the longitudinal axis of the drug delivery device. First and second pattern portions 110, 112 are arranged non-overlapping on the tubular-shaped surface of the skirt 6. These two pattern portions are suitable for detecting e.g. a rotation of the skirt 6 relative to the outer housing 1. Further pattern portions 114, 116 may be provided to detect additional movements, e.g. reaching an end position of the skirt 6 relative to the outer housing 1.

Optionally, the first and the second pattern portions 110, 112 may also distinguish from each other with regard to a radial position with regard to a central axis of the tubular-shaped skirt 6. For instance, the first pattern portion 110 may comprise one or several radial protrusions and the second pattern portion 112 may comprise one or several radial indentations. The radial protrusions and indentations may be provided on an outer tubular-shaped surface of the skirt 6.

At least one electric sensor is provided by the contact tabs 164, 166 and 168 in combination with the conductive strips 12, 13, 14. The at least one electric sensor is configured to distinguish between the first pattern portion 110 and the second pattern portion 112 if the respective pattern portions 110, 112 are in a defined region of coverage of the at least one electric sensor. As the first pattern portion 110 distinguishes by its electrical conductivity from the second pattern portion 112, the at least one electric sensor comprising the electric contact tabs 164, 166 and 168 allow to generate varying electric signals as first and second pattern portions 110, 112 pass by the electric contact tabs 164, 166 and 168.

As mentioned above, the pattern is not limited to only a first pattern portion 110 and a second pattern portion 112. There may be provided also a third pattern portion 114 and a fourth pattern portion 116 as shown in Figure 3 and numerous further pattern portions that distinguish from each other, thus allowing to implement not only a 2-bit pattern but supporting a 3-bit, 4-bit, 5-bit, 6-bit or even n-bit pattern, with n being an integer number larger than 0. The type of pattern, hence the type of first and second pattern portions 110, 112 provided in the first pattern section 120 and provided in the second pattern section 122 may be substantially equal. However, the pattern and the pattern portions 110, 112 of the first pattern section 120, in particular their geometry, shape and orientation may distinguish from respective pattern portions 110, 112 provided in the second pattern section 122.

The working principle of this 2-bit encoding with a supplemental zero dose position detection is based on the bridging contact 15 being fixedly connected to the outer housing 1 and arranged at an inside tubular-shaped sidewall of the housing 1 or the retention cap 11. As illustrated in more detail in Figure 4, the bridging contact 15 comprises three contact tabs 164, 166, 168 located at a longitudinal and hence at a free end of respective flexible arms. All arms as well as the respective contact tabs 164, 166, 168 are electrically interconnected. The bridging contact 15 may comprise a piece of sheet metal.

As schematically illustrated the first contact tap 164 and the second contact tap 166 are separated from each other along a first separation direction D1. The first separation direction D1 may coincide with the tangential or circumferential direction of the tubular-shaped pattern. The first and the second contact tabs 164, 166 may be arranged at the same longitudinal position. The third contact tap 168 is located at a longitudinal distance from at least one of the first and second contact tabs 164, 166. It is typically separated in longitudinal direction along the second separation direction D2 from both of the first and the second contact tabs 164, 166. The contact tabs 164, 166, 168 are integrally formed in the flexible arms and may each comprise radially inwardly protruding embossed portions. The contact tabs 164, 166, 168 may comprise a partially dome-shaped structure and may thus protrude radially inwardly from the rather planar-shaped surface of the flexible arms. In this way, and due to the non-negligible radial protrusion of the contact tabs 164, 166, 168 there may be provided a good and reliable mechanical and hence electrical contact between the contact tabs 164, 166, 168 and the electrically conductive structures, hence with numerous pattern portions 110, 114, 116 of the pattern of the skirt 6. Typically, the flexible arms and the radial protrusion of the respective contact tabs 164, 166, 168 provide a kind of a radial preload as the skirt 6 is arranged inside the hollow tubular-shaped outer housing 1 or the retention cap 11.

During dose setting and dose dispensing, the skirt 6 of the dose dial grip 4 is subject to a sliding motion relative to the outer housing 1 and hence relative to the bridging contact 15. In the configuration of Figures 1, 2 and 6, the skirt 6 is located close to a zero dose position but has not yet reached this zero dose position. Here, the first pattern portion 110 is in electrical contact with the third pattern portion 114 via the bridging contact 15. In this configuration the third contact tap 168 is located in the first pattern section 120. Hence, it is located distally from the separation 121.

Now, and as the skirt 6 approaches and reaches the zero dose configuration relative to the outer housing 1 and hence relative to the reference element the third contact tap 168 has traversed the separation 121 and comes into electrical contact with the fourth pattern portion 116 while the first contact tap 164 remains in electrical contact with the third pattern portion 114. In this way, the supply voltage present on the third pattern portion 114 is provided also to the fourth pattern portion 116. The input terminal of the detector arrangement is then tied to the supply voltage and switches from a logical 0 to a logical 1 as the zero dose configuration has been reached. In this way, the zero dose position of the skirt 6 relative to the outer housing 1 can be precisely detected at the end of a dose dispensing procedure.

The core encoding principle as depicted in the embodiment of Figures 1 to 6 is based on a series of conductive strips 12, 13, 14 formed on the outside diameter of the dose dial grip 4. The rotation of this component is encoded by electrically connecting the conductive strips 12, 13, 14 to the input of a microcontroller 21 by means of the connectors 22 of the module 20. Bridging contacts 164, 166, 168 mounted e.g. in the housing 1 selectively connect and disconnect conductive strips 12, 13, 14 on the dose dial grip 4, thereby alternating the signals received by the microcontroller.

The present disclosure relates to the combination of the electronic module 20 that can be embodied as a re-usable clip-on module with a suitably configured pen injector or drug delivery device for the purpose of recording doses that are dialed and delivered from the pen. This functionality may be of value to a wide variety of device users as a memory aid or to support detailed logging of dose history. It is possible that the module 20 is configured to be connectable to a mobile device, or similar, to enable the dose history to be downloaded from the module 20 on a periodic basis.

When the drug delivery device is operated without the addition of the clip-on module 20, the conductive strips 12, 13, 14 are passive. The clip-on module 20 connects electrically to these conductive strips 12, 13, 14 when fitted.

In summary, the only additional elements required for a standard drug delivery device are three bridging contacts 164, 166, 168, conductive printing 12, 13, 14 on the dose dial grip 4 and, optionally, a retention cap 11. In certain markets where clip-on connectivity will not be required; it is possible to assemble devices without the bridging contacts or conductive printing.

A second embodiment is depicted in Figures 7 and 8. Again, the drug delivery device is provided with a dose setting and driving assembly comprising an outer housing 1, a number sleeve 3 and a dose dial grip 4. Instead of the conductive strips 12, 13, 14 on the dose dial grip 4, the number sleeve 3 is provided with an encoder wheel 29 having conductive and non conductive portions (contact/non-contact areas) distributed about the outer circumference of the encoder wheel 29. As shown in Figures 7 and 8, the encoder wheel 29 may be arranged at the proximal end of the number sleeve 3, for example proximally of the clutch teeth 9. Further, instead of the bridging contacts 15 at least two brushing contacts 30 are fixedly arranged as reference elements to enable quadrature encoding (direction detection) of the number sleeve 3.

The module 20 can be attached to and detached from the dose dial grip 4 in a similar manner as explained above with respect to the first embodiment. The module 20 comprises all electronic components, e.g. a battery 24, a processor 21, a transceiver, like NFC, BT, etc. (not shown) as well as the housing 25, an optional end cap 26 and a battery carrier 28. The interface between the drug delivery device and the module 20 comprises mechanical fixation means (e.g. snap in features) as described above. Electrical connection means may be provided between the module 20 and the brushing contacts 30, e.g. and the form of spring contacts.

Thus, in a similar manner as described with respect to the first embodiment, a drug delivery device may be equipped with low-cost "sensing" features, e.g. the encoder wheel 29 and the brushing contacts 30, such that the reusable clip-on electronic module 20 can be easily attached when desired, for example either at the production side or later by a HCP or a user.

### Reference Numerals

| | | | |
|---|---|---|---|
| 1 | (outer) housing | 27 | alignment feature |
| 2 | cartridge holder | 28 | battery carrier |
| 3 | number sleeve | 29 | encoder wheel |
| 4 | dose dial grip | 30 | brushing contact |
| 5 | aperture | | |
| 6 | skirt | 110 | first pattern portion |
| 7 | profiled gripping surface | 112 | second pattern portion |
| 8 | driver | 114 | third pattern portion |
| 9 | clutch (teeth) | 116 | fourth pattern portion |
| 10 | stem | | |
| 11 | retention cap | 120 | first pattern section |
| 12 | conductive strip | 121 | separation |
| 13 | conductive strip | 122 | second pattern section |
| 14 | conductive strip | | |
| 15 | bridging contact (reference element) | 164 | contact tab |
| 20 | module | 166 | contact tab |
| 21 | microprocessor | 168 | contact tab |
| 22 | connector | | |
| 23 | clip feature | D1 | first direction |
| 24 | coin cell (battery) | D2 | second direction |
| 25 | housing | | |
| 26 | end cap | l | longitudinal axis |

## Claims

1. A drug delivery device, comprising a dose setting and driving assembly for dialing and dispensing a dose of a medicament from a cartridge of the drug delivery device, the dose setting and driving assembly comprising an outer housing (1, 11), a number sleeve (3) and a dose dial grip (4), wherein a portion of the number sleeve (3) is visible through an aperture or window (5) in the outer housing (1,11) to display the actually dialed dose, wherein the number sleeve (3) and the dose dial grip (4) are rotatable relative to the outer housing (1, 11) during dose dialing and wherein the number sleeve (3) is rotatable relative to the outer housing (1, 11) during dose dispensing, while the dose dial grip (4) is axially displaceable and rotationally constrained to the outer housing (1, 11) during dose dispensing, wherein the dose dial grip (4) comprises an outer skirt (6) partially protruding out of the outer housing (1, 11), **characterized in that** at least one of the number sleeve (3) and the skirt (6) of the dose dial grip (4) is provided with a series of circumferentially spaced conductive strips (12, 13, 14; 29) distributed around the outer circumference of the number sleeve (3) or the skirt (6) to provide at least two different pattern portions (110, 112, 114, 116) which are electrically separated, and that a series of contacts (164, 166, 168; 30) is arranged within the outer housing (1, 11) biased to abut the conductive strips, wherein the series of contacts (164, 166, 168; 30) is part of at least one bridging contact (15) in the drug delivery device configured and operable to selectively establish an electric connection between the at least two different pattern portions (110, 112, 114, 116) as number sleeve (3) and/or the skirt (6) is subject to a helical or rotational movement relative to the outer housing (1, 11), wherein the conductive strips (12, 13, 14; 29) and/or the contacts (15; 164, 166, 168; 30) are directly accessible from outside the outer housing (1, 11).

2. The drug delivery device according to claim 1, wherein the series of contacts (164, 166, 168) form at least one reference element (15; 30), wherein the conductive strips (12, 13, 14; 29) form a pattern, and wherein the contacts and the conductive strips together form a detector arrangement comprising at least one electric sensor being operable to detect a positional variation of the pattern relative to the at least one reference element (15; 30) and to generate at least one electric signal in response to the positional variation of the pattern during at least one of the movements of the number sleeve (3) and/or the skirt (6) during dose dialing and dose dispensing relative to the outer housing (1, 11).

3. The drug delivery device according to claim 2, wherein the pattern comprises at least a first pattern portion (110) that is electrically conductive and at least a second pattern portion (112) that is electrically insulating.

4. The drug delivery device according to claim 3, wherein the contacts (164, 166, 168; 30) of the detector arrangement are operable to alternately connect to the first pattern portion (110) and the second pattern portion (112) of the pattern during at least one of the movements of the number sleeve (3) and/or the skirt (6) during dose dialing and dose dispensing relative to the outer housing (1, 11).

5. The drug delivery device according to claim 3 or 4, wherein the pattern comprises at least a third pattern portion (114) that is electrically conductive, wherein the first pattern portion (110) and the third pattern portion (114) are electrically separated.

6. The drug delivery device according to claim 5, wherein the detector arrangement and the at least one electric sensor are arranged on the outer circumference of the number sleeve (3) or the skirt (6), wherein the at least one electric sensor is electrically connected to the first pattern portion (110) and wherein the at least one reference element (15; 30) is arranged on the outer housing (1, 11) and comprises an electrical bridging contact (15) configured to alternately establish and interrupt an electric contact between the first pattern portion (110) and the third pattern portion (114) during at least one of the movements of the number sleeve (3) and/or the skirt (6) during dose dialing and dose dispensing relative to the outer housing (1, 11).

7. The drug delivery device according to claim 6, wherein the electrical bridging contact (15) comprises a first electrical contact tap (164) and a second electrical contact tap (166), wherein the first electrical contact tap (164) and the second electrical contact tap (166) are electrically connected and wherein the first electrical contact tap (164) and the second electrical contact tap (166) are spatially separated from each other along a first separation direction (D1) parallel to a distance between the first pattern portion (110) and the third pattern portion (114).

8. The drug delivery device according to claim 7, wherein the electrical bridging contact (15) comprises a third electrical contact tap (168) spatially separated from at least one of the first electrical contact tap (164) and the second electrical contact tap (166) along a second separation direction (D2) non-parallel to the first separation direction (D1).

9. The drug delivery device according to any one of claims 6 to 8, wherein the electrical bridging contact (15) comprises a body made of sheet-metal and comprising at least one flexible arm, wherein at least one of the first electrical contact tap (164) and the second electrical contact tap (166) is arranged at a free end of the at least one flexible arm.

10. The drug delivery device according to any one of the preceding claims, wherein the conductive strips (12, 13, 14) are printed on the skirt (6) of the dose dial grip (4).

11. The drug delivery device according to any one of claims 1 to 9, wherein the conductive strips are provided on an encoder wheel (29) fixed to the number sleeve (3).

12. The drug delivery device according to any one of the preceding claims, wherein the dose dial grip (4) comprises mechanical fixation means, e.g. clip features (23), and/or rotational alignment means (27) for attaching a separate module (20) on the dose dial grip (4).

13. A module for detecting movement of at least one component part of a drug delivery device according to any one of the preceding claims, wherein the module (20) comprises
- a microprocessor (21) for processing and/or storing data from a detector arrangement of the drug delivery device,
- electrical connectors (22) to releasably establish contact with conductive strips (12, 13, 14) or contacts (30) of the drug delivery device, wherein the electrical connectors (22) comprise metal clips having a free end biased radially inwards for abutting the conductive strips (12, 13, 14) or contacts (30) of the drug delivery device, and
- mechanical fixation means, e.g. clip features (23), and/or rotational alignment means (27) for attaching the module (20) on the dose dial grip (4) of the drug delivery device.

14. The module according to claim 13, further comprising a cap (25, 26) to be, e.g. releasably, fitted over the dose dial grip (4) of the drug delivery device, wherein the cap (25, 26) comprises an outer profiled gripping surface and encloses the microprocessor (21) and the electrical connectors (22).

## Patentansprüche

1. Medikamenten-Verabreichungsvorrichtung, umfassend eine Dosiseinstellungs- und Antriebsanordnung zum Wählen und Abgeben einer Dosis eines Arzneimittels aus einer Kartusche der Medikamenten-Verabreichungsvorrichtung, wobei die Dosiseinstellungs- und Antriebsanordnung ein Außengehäuse (1, 11), eine Zahlenhülse (3) und einen Dosiswahlgriff (4) umfasst, wobei ein Abschnitt der Zahlenhülse (3) durch eine Öffnung oder ein Fenster (5) in dem Außengehäuse (1, 11) sichtbar ist, um die tatsächlich gewählte Dosis anzuzeigen, wobei die Zahlenhülse (3) und der Dosiswahlgriff (4) während einer Dosiswahl relativ zu dem Außengehäuse (1, 11) drehbar sind und wobei die Zahlenhülse (3) während einer Dosisabgabe relativ zu dem Außengehäuse (1, 11) drehbar ist, während der Dosiswahlgriff (4) während der Dosisabgabe axial verschiebbar und an dem Außengehäuse (1, 11) drehfest ist, wobei der Dosiswahlgriff (4) eine Außenschürze (6) umfasst, die teilweise aus dem Außengehäuse herausragt (1, 11), **dadurch gekennzeichnet, dass** mindestens eines der Zahlenhülse (3) und der Schürze (6) des Dosiswahlgriffs (4) mit einer Reihe von in Umfangsrichtung beabstandeten leitfähigen Streifen (12, 13, 14; 29) versehen ist, die um den Außenumfang der Zahlenhülse (3) oder der Schürze (6) verteilt sind, um mindestens zwei verschiedene Musterabschnitte (110, 112, 114, 116) bereitzustellen, die elektrisch getrennt sind, und dass eine Reihe von Kontakten (164, 166, 168; 30) innerhalb des Außengehäuses (1, 11) angeordnet ist, die so vorgespannt sind, dass sie an den leitfähigen Streifen anliegen, wobei die Reihe von Kontakten (164, 166, 168; 30) ein Teil mindestens eines Überbrückungskontakts (15) in der Medikamenten-Verabreichungsvorrichtung ist, der dazu ausgelegt und betreibbar ist, selektiv eine elektrische Verbindung zwischen den mindestens zwei unterschiedlichen Musterabschnitten (110, 112, 114, 116) herzustellen, während die Zahlenhülse (3) und/oder die Schürze (6) einer Spiral- oder Drehbewegung relativ zu dem Außengehäuse (1, 11) unterzogen wird, wobei die leitfähigen Streifen (12, 13, 14; 29) und/oder die Kontakte (15; 164, 166, 168; 30) direkt von außerhalb des Außengehäuses (1, 11) zugänglich sind.

2. Medikamenten-Verabreichungsvorrichtung nach Anspruch 1, wobei die Reihe von Kontakten (164, 166, 168) mindestens ein Bezugselement (15; 30) bilden, wobei die leitfähigen Streifen (12, 13, 14; 29) ein Muster bilden, und wobei die Kontakte und die leitfähigen Streifen zusammen eine Detektoranordnung bilden, die mindestens einen elektrischen Sensor umfasst, der dazu betreibbar ist, eine Positionsänderung des Musters relativ zu dem mindestens einen Bezugselement (15; 30) zu detektieren und mindestens ein elektrisches Signal als Reaktion auf die Positionsänderung des Musters während mindestens einer der Bewegungen der Zahlenhülse (3) und/oder der Schürze (6) während der Dosiswahl und der Dosisabgabe relativ zu dem Außengehäuse (1, 11) zu erzeugen.

3. Medikamenten-Verabreichungsvorrichtung nach Anspruch 2, wobei das Muster mindestens einen ersten Musterabschnitt (110), der elektrisch leitfähig ist, und mindestens einen zweiten Musterabschnitt (112), der elektrisch isolierend ist, umfasst.

4. Medikamenten-Verabreichungsvorrichtung nach Anspruch 3, wobei die Kontakte (164, 166, 168; 30) der Detektoranordnung dazu betreibbar sind, während mindestens einer der Bewegungen der Zahlenhülse (3) und/oder der Schürze (6) während der Dosiswahl und der Dosisabgabe relativ zu dem Außengehäuse (1, 11) abwechselnd mit dem ersten Musterabschnitt (110) und dem zweiten Musterabschnitt (112) des Musters verbunden zu werden.

5. Medikamenten-Verabreichungsvorrichtung nach Anspruch 3 oder 4, wobei das Muster mindestens einen dritten Musterabschnitt (114) umfasst, der elektrisch leitfähig ist, wobei der erste Musterabschnitt (110) und der dritte Musterabschnitt (114) elektrisch getrennt sind.

6. Medikamenten-Verabreichungsvorrichtung nach Anspruch 5, wobei die Detektoranordnung und der mindestens eine elektrische Sensor an dem Außenumfang der Zahlenhülse (3) oder der Schürze (6) angeordnet sind, wobei der mindestens eine elektrische Sensor elektrisch mit dem ersten Musterabschnitt (110) verbunden ist und wobei das mindestens eine Bezugselement (15; 30) an dem Außengehäuse (1, 11) angeordnet ist und einen elektrischen Überbrückungskontakt (15) umfasst, der dazu ausgelegt ist, während mindestens einer der Bewegungen der Zahlenhülse (3) und/oder der Schürze (6) während der Dosiswahl und der Dosisabgabe relativ zu dem Außengehäuse (1, 11) abwechselnd einen elektrischen Kontakt zwischen dem ersten Musterabschnitt (110) und dem dritten Musterabschnitt (114) herzustellen und zu unterbrechen.

7. Medikamenten-Verabreichungsvorrichtung nach Anspruch 6, wobei der elektrische Überbrückungskontakt (15) einen ersten elektrischen Kontaktabgriff (164) und einen zweiten elektrischen Kontaktabgriff (166) umfasst, wobei der erste elektrische Kontaktabgriff (164) und der zweite elektrische Kontaktabgriff (166) elektrisch verbunden sind und wobei der erste elektrische Kontaktabgriff (164) und der zweite elektrische Kontaktabgriff (166) entlang einer ersten Trennrichtung (D1) parallel zu einem Abstand zwischen dem ersten Musterabschnitt (110) und dem dritten Musterabschnitt (114) räumlich voneinander getrennt sind.

8. Medikamenten-Verabreichungsvorrichtung nach Anspruch 7, wobei der elektrische Überbrückungskontakt (15) einen dritten elektrischen Kontaktabgriff (168) umfasst, der entlang einer zweiten Trennrichtung (D2), die nicht parallel zu der ersten Trennrichtung (D1) ist, von mindestens einem des ersten elektrischen Kontaktabgriffs (164) und des zweiten elektrischen Kontaktabgriffs (166) räumlich getrennt ist.

9. Medikamenten-Verabreichungsvorrichtung nach einem der Ansprüche 6 bis 8, wobei der elektrische Überbrückungskontakt (15) einen Körper umfasst, der aus Blech besteht und mindestens einen flexiblen Arm umfasst, wobei mindestens einer des ersten elektrischen Kontaktabgriffs (164) und des zweiten elektrischen Kontaktabgriffs (166) an einem freien Ende des mindestens einen flexiblen Arms angeordnet ist.

10. Medikamenten-Verabreichungsvorrichtung nach einem der vorhergehenden Ansprüche, wobei die leitfähigen Streifen (12, 13, 14) auf die Schürze (6) des Dosiswahlgriffs (4) gedruckt sind.

11. Medikamenten-Verabreichungsvorrichtung nach einem der Ansprüche 1 bis 9, wobei die leitfähigen Streifen auf einem Encoderrad (29) bereitgestellt sind, das an der Zahlenhülse (3) fixiert ist.

12. Medikamenten-Verabreichungsvorrichtung nach einem der vorhergehenden Ansprüche, wobei der Dosiswahlgriff (4) mechanische Fixierungsmittel, z. B. Klemmmerkmale (23), und/oder Drehausrichtungsmittel (27) zum Befestigen eines separaten Moduls (20) an dem Dosiswahlgriff (4) umfasst.

13. Modul zum Detektieren einer Bewegung mindestens eines Komponententeils einer Medikamenten-Verabreichungsvorrichtung nach einem der vorhergehenden Ansprüche, wobei das Modul (20) Folgendes umfasst
- einen Mikroprozessor (21) zum Verarbeiten und/oder Speichern von Daten von einer Detektoranordnung der Medikamenten-Verabreichungsvorrichtung,
- elektrische Verbinder (22) zum lösbaren Herstellen eines Kontakts mit leitfähigen Streifen (12, 13, 14) oder Kontakten (30) der Medikamenten-Verabreichungsvorrichtung, wobei die elektrischen Verbinder (22) Metallklemmen umfassen, die ein freies Ende aufweisen, das radial nach innen vorgespannt ist, um an den leitfähigen Streifen (12, 13, 14) oder Kontakten (30) der Medikamenten-Verabreichungsvorrichtung anzuliegen, und
- mechanische Fixierungsmittel, z. B. Klemmmerkmale (23), und/oder Drehausrichtungsmittel (27) zum Befestigen des Moduls (20) an dem Dosiswahlgriff (4) der Medikamenten-Verabreichungsvorrichtung.

14. Modul nach Anspruch 13, ferner umfassend eine Kappe (25, 26) zum, z. B. lösbaren, Anbringen über dem Dosiswahlgriff (4) der Medikamenten-Verabreichungsvorrichtung, wobei die Kappe (25, 26) eine äußere profilierte Grifffläche umfasst und den Mikroprozessor (21) und die elektrischen Verbinder (22) umschließt.

## Revendications

1. Dispositif d'administration de médicament, comprenant un ensemble de réglage et d'entraînement de dose pour composer et distribuer une dose d'un médicament à partir d'une cartouche du dispositif d'administration de médicament, l'ensemble de réglage et d'entraînement de dose comprenant un boîtier externe (1, 11), un manchon numérique (3) et une poignée de sélection de dose (4), une partie du manchon numérique (3) étant visible à travers une ouverture ou une fenêtre (5) dans le boîtier externe (1, 11) pour afficher la dose effectivement composée, le manchon numérique (3) et la poignée de sélection de dose (4) pouvant tourner par rapport au boîtier externe (1, 11) pendant la sélection de dose, et le manchon numérique (3) pouvant tourner par rapport au boîtier externe (1, 11) pendant la distribution de dose, tandis que la poignée de sélection de dose (4) peut coulisser axialement et est bloquée en rotation par rapport au boîtier externe (1, 11) pendant la distribution de dose, la poignée de sélection de dose (4) comprenant une jupe externe (6) partiellement en saillie hors du boîtier externe (1, 11), **caractérisé en ce qu'**au moins l'un du manchon numérique (3) et de la jupe (6) de la poignée de sélection de dose (4) est pourvu d'une série de bandes conductrices (12, 13, 14 ; 29) espacées circonférentiellement réparties sur la circonférence extérieure du manchon numérique (3) ou de la jupe (6) pour fournir au moins deux parties de motif différentes (110, 112, 114, 116) séparées électriquement, et qu'une série de contacts (164, 166, 168 ; 30) est agencée à l'intérieur du boîtier externe (1, 11) sollicitée pour buter contre les bandes conductrices, la série de contacts (164, 166, 168 ; 30) faisant partie d'au moins un contact de pontage (15) dans le dispositif d'administration de médicament configuré et apte à établir sélectivement une connexion électrique entre les au moins deux parties de motif différentes (110, 112, 114, 116) lorsque le manchon numérique (3) et/ou la jupe (6) est soumis(e) à un mouvement hélicoïdal ou rotatif par rapport au boîtier externe (1, 11), les bandes conductrices (12, 13, 14 ; 29) et/ou les contacts (15 ; 164, 166, 168 ; 30) étant directement accessibles depuis l'extérieur du boîtier externe (1, 11).

2. Dispositif d'administration de médicament selon la revendication 1, la série de contacts (164, 166, 168) formant au moins un élément de référence (15 ; 30), les bandes conductrices (12, 13, 14 ; 29) formant un motif, et les contacts et les bandes conductrices formant ensemble un agencement de détecteur comprenant au moins un capteur électrique pouvant fonctionner pour détecter une variation de position du motif par rapport à l'au moins un élément de référence (15 ; 30) et pour générer au moins un signal électrique en réponse à la variation de position du motif pendant au moins un des mouvements du manchon numérique (3) et/ou de la jupe (6) pendant la sélection de dose et la distribution de dose par rapport au boîtier externe (1, 11).

3. Dispositif d'administration de médicament selon la revendication 2, le motif comprenant au moins une première partie de motif (110) qui est électriquement conductrice et au moins une deuxième partie de motif (112) qui est électriquement isolante.

4. Dispositif d'administration de médicament selon la revendication 3, les contacts (164, 166, 168 ; 30) de l'agencement de détecteur pouvant être actionnés pour se connecter en alternance à la première partie de motif (110) et à la deuxième partie de motif (112) du motif pendant au moins l'un des mouvements du manchon numérique (3) et/ou de la jupe (6) pendant la sélection de dose et la distribution de dose par rapport au boîtier externe (1, 11).

5. Dispositif d'administration de médicament selon la revendication 3 ou 4, le motif comprenant au moins une troisième partie de motif (114) qui est électriquement conductrice, la première partie de motif (110) et la troisième partie de motif (114) étant électriquement séparées.

6. Dispositif d'administration de médicament selon la revendication 5, l'agencement de détecteur et l'au moins un capteur électrique étant agencés sur la circonférence extérieure du manchon numérique (3) ou de la jupe (6), l'au moins un capteur électrique étant connecté électriquement à la première partie de motif (110), et l'au moins un élément de référence (15 ; 30) étant agencé sur le boîtier externe (1, 11) et comprenant un contact de pontage électrique (15) configuré pour établir et interrompre en alternance un contact électrique entre la première partie de motif (110) et la troisième partie de motif (114) pendant au moins un des mouvements du manchon numérique (3) et/ou de la jupe (6) pendant la sélection de dose et la distribution de dose par rapport au boîtier externe (1, 11).

7. Dispositif d'administration de médicament selon la revendication 6, le contact de pontage électrique (15) comprenant une première prise de contact électrique (164) et une deuxième prise de contact électrique (166), la première prise de contact électrique (164) et la deuxième prise de contact électrique (166) étant électriquement connectées et la première prise de contact électrique (164) et la deuxième prise de contact électrique (166) étant spatialement séparées l'une de l'autre le long d'une première direction de séparation (D1) parallèle à une distance entre la première partie de motif (110) et la troisième partie de motif (114).

8. Dispositif d'administration de médicament selon la revendication 7, le contact de pontage électrique (15) comprenant une troisième prise de contact électrique (168) spatialement séparée d'au moins l'une de la première prise de contact électrique (164) et de la deuxième prise de contact électrique (166) le long d'une deuxième direction de séparation (D2) non parallèle à la première direction de séparation (D1).

9. Dispositif d'administration de médicament selon l'une quelconque des revendications 6 à 8, le contact de pontage électrique (15) comprenant un corps réalisé en tôle métallique et comprenant au moins un bras flexible, au moins l'une de la première prise de contact électrique (164) et de la deuxième prise de contact électrique (166) étant agencée à une extrémité libre de l'au moins un bras flexible.

10. Dispositif d'administration de médicament selon l'une quelconque des revendications précédentes, les bandes conductrices (12, 13, 14) étant imprimées sur la jupe (6) de la poignée de sélection de dose (4).

11. Dispositif d'administration de médicament selon l'une quelconque des revendications 1 à 9, les bandes conductrices étant prévues sur une roue codeuse (29) fixée au manchon numérique (3).

12. Dispositif d'administration de médicament selon l'une quelconque des revendications précédentes, la poignée de sélection de dose (4) comprenant des moyens de fixation mécaniques, par exemple des éléments de clip (23), et/ou des moyens d'alignement en rotation (27) pour fixer un module séparé (20) sur la poignée de sélection de dose (4).

13. Module pour détecter le mouvement d'au moins une partie constitutive d'un dispositif d'administration de médicament selon l'une quelconque des revendications précédentes, le module (20) comprenant
- un microprocesseur (21) pour le traitement et/ou le stockage de données issues d'un agencement de détecteur du dispositif d'administration de médicament,
- des connecteurs électriques (22) pour établir un contact amovible avec des bandes conductrices (12, 13, 14) ou des contacts (30) du dispositif d'administration de médicament, les connecteurs électriques (22) comprenant des clips métalliques dont l'extrémité libre est sollicitée radialement vers l'intérieur pour venir buter contre les bandes conductrices (12, 13, 14) ou les contacts (30) du dispositif d'administration de médicament, et
- des moyens de fixation mécaniques, par exemple des éléments de clip (23), et/ou des moyens d'alignement en rotation (27) pour fixer le module (20) sur la poignée de sélection de dose (4) du dispositif d'administration de médicament.

14. Module selon la revendication 13, comprenant en outre un capuchon (25, 26) destiné à être, par exemple de manière amovible, monté sur la poignée de sélection de dose (4) du dispositif d'administration de médicament, le capuchon (25, 26) comprenant une surface de préhension profilée extérieure et enfermant le microprocesseur (21) et les connecteurs électriques (22).
